# EUROPÄISCHE PATENTANMELDUNG

(11) **EP 3 915 400 A1**
(43) Veröffentlichungstag der Anmeldung: **01.12.2021**
(21) Anmeldenummer: 20176231.7
(22) Anmeldetag: 25.05.2020
(51) Int. Cl.: A23L 33/125, A23C 9/142, A23C 9/144, A23C 9/146, A23C 9/20, A23C 21/02, A23G 3/42, A23G 4/10, A23L 27/30, A23L 29/30, A23L 33/20, A61K 31/7016, C07H 1/08

(54) **LACTOSEDERIVAT MIT ERHÖHTER SÜSSKRAFT**

(71) Anmelder: DMK Deutsches Milchkontor GmbH, 27404 Zeven (DE)
(72) Erfinder: DÖRING, Sven-Rainer, 27404 Zeven (DE); STEFFENS, Marco, 27404 Elsdorf (DE)
(74) Vertreter: Fabry, Bernd

(57) **Zusammenfassung**

Vorgeschlagen wird ein Lactosederivat mit erhöhter Süßkraft, dadurch erhältlich oder erhalten, dass man
(a) eine lactosehaltige Milchfraktion bereitstellt;
(b) die Milchfraktion einer Ultrafiltration unterwirft und dabei ein erstes protein- und fettreiches Retentat R1 und ein erstes lactosehaltiges Permeat P1 erhält;
(c) das erste Permeat P1 einer Umkehrosmose unterwirft und dabei ein lactoseangereichertes zweites Retentat R2 und Permeatwasser erhält;
(d) das zweite Retentat R2
(d1) einer Elektrodialyse und/oder
(d2) einer Kationenaustauscherbehandlung
unterwirft und dabei eine Salzfraktion und als Diluat eine weiter aufkonzentrierte Lactoselösung erhält;

(e) die aufkonzentrierte Lactoselösung in einer ersten Enzymbehandlungsstufe mit Lactase versetzt und die Lactose vollständig oder zumindest annähernd vollständig zu einem Gemisch von Glucose und Galactose umsetzt;
(f) die so erhaltene Glucose/Galactose-Lösung in einer zweiten Enzymbehandlungsstufe mit Glucoseisomerase versetzt und dabei einen Teil der Glucose in Fructose umlagert; sowie gegebenenfalls
(g) das so erhaltene Endprodukt entwässert.

## Beschreibung

### GEBIET DER ERFINDUNG

Die Erfindung befindet sich auf dem Gebiet der Molkereiindustrie und betrifft einen milchbasierten Lactosederivat mit erhöhter Süßkraft, ein Verfahren zu seiner Herstellung sowie seine Verwendung.

### TECHNOLOGISCHER HINTERGRUND

Einer Studie des Robert-Koch Instituts aus dem Jahre 2014 nach sind zwei Drittel der Männer (67 %) und die Hälfte der Frauen (53 %) in Deutschland übergewichtig. Ein Viertel der Erwachsenen (23 % der Männer und 24 % der Frauen) ist stark übergewichtig (adipös). Bei den Jugendlichen ist inzwischen jeder und bei den Kindern jedes fünfte übergewichtig. Das sind mehr als dreieinhalb Millionen Jungen und Mädchen unter 18. In die gleiche Richtung weist der zunehmende Verzehr von Fast Food, ebenfalls verstärkt bei Jugendlichen.

Übergewicht und Adipositas sind Mitursache für viele Beschwerden und können die Entwicklung chronischer Krankheiten begünstigen. Aufgrund der steigenden Prävalenz und den damit verbundenen Folgeerkrankungen entstehen beträchtliche Kosten für das Gesundheits- und Sozialsystem. Übergewicht und Adipositas sind daher Themen von hoher Public-Health-Relevanz.

Maßgeblich für diese Situation ist das Essverhalten in Kombination mit sitzender Tätigkeit und dem Mangel an Sport. Ein Ansatz besteht sicher darin, über die gesundheitlichen Risiken im Zusammenhang mit diesen Essgewohnheiten hinzuweisen; eine durchgreifende Änderung wird dadurch jedoch wohl kaum zu erreichen sein. Wer seine zuckerhaltige Cola liebt, wird kaum bereit sein, stattdessen zu einem Broccoli-Smoothie zu greifen. Es besteht daher ein großes Marktinteresse an Austauschstoffen insbesondere für Milchzucker, die eine deutlich höhere Süßkraft aufweisen, so dass die Einsatzmengen reduziert und entsprechend die damit verbundene Kalorienaufnahme verringert werden kann.

### RELEVANTER STAND DER TECHNIK

Aus der EP 3251515 A1 (DMK) ist ein Verfahren zur Herstellung von lactosefreien Milchprodukten bekannt, das die folgenden Schritte umfasst: (a) Ultrafiltration einer Ausgangsmilch zur Erzeugung eines ersten Permeats P1 und eines ersten Retentats R1; (b) Umkehrosmose des ersten Permeats P1 zur Erzeugung eines zweiten Permeats P2 und eines zweiten Retentats R2; (c) Vermischen des ersten Retentats R1 mit jeweils einer solchen Menge des zweiten Permeats P2 und Milchmineralien, so dass ein standardisiertes Milchprodukt erhalten wird, dessen Gehalt an Proteinen und Mineralien dem der Ausgangsmilch annähernd entspricht, sowie (d) Hydrolyse des standardisierten Milchproduktes aus Schritt (e) unter Zugabe einer solchen Menge an Lactase, dass die noch im Produkt enthaltene Restmenge an Lactose vollständig in Glucose und Galactose gespalten wird.

### AUFGBE DER ERFINDUNG

Die Aufgabe der vorliegenden Erfindung hat daher darin bestanden, ein natürliches, konkret ein auf Milch basiertes Lactosederivat zur Verfügung zu stellen, das über mindestens die doppelte Süßkraft von Lactose verfügt und das daher insbesondere in Nahrungsmitteln eingesetzt werden kann. Auf diese Weise sollen orale Zubereitungen zur Verfügung gestellt werden, die bei gleicher Süßkraft deutlich weniger Kohlenhydrate enthalten und daher entsprechend kalorienärmer sind.

Außerdem sollten die Lactosederivate insbesondere mit pflanzlichen Süßstoffen und Süßstoffverstärkern kombinierbar sein und vorzugsweise deren Eigenschaften verstärken oder Schwächen maskieren.

### BESCHREIBUNG DER ERFINDUNG

Ein erster Gegenstand der Erfindung betrifft ein Lactosederivat mit erhöhter Süßkraft, da dadurch erhältlich ist oder erhalten wird, dass man
(a) eine lactosehaltige Milchfraktion bereitstellt;
(b) die Milchfraktion einer Ultrafiltration und/oder Nanofiltration unterwirft und dabei ein erstes protein- und fettreiches Retentat R1 und ein erstes lactosehaltiges Permeat P1 erhält;
(c) das erste Permeat P1 einer Umkehrosmose unterwirft und dabei ein lactoseangereichertes zweites Retentat R2 und Permeatwasser erhält;
(d) das zweite Retentat R2
   (d1) einer Elektrodialyse und/oder
   (d2) einer Kationenaustauscherbehandlung
   unterwirft und dabei eine Salzfraktion und als Diluat eine weiter aufkonzentrierte Lactoselösung erhält;
(e) die aufkonzentrierte Lactoselösung in einer ersten Enzymbehandlungsstufe mit Lactase versetzt und die Lactose vollständig oder zumindest annähernd vollständig zu einem Gemisch von Glucose und Galactose umsetzt;
(f) die so erhaltene Glucose/Galactose-Lösung in einer zweiten Enzymbehandlungsstufe mit Glucoseisomerase versetzt und dabei einen Teil der Glucose in Fructose umlagert; sowie gegebenenfalls
(g) das so erhaltene Endprodukt entwässert.

Ein weiterer Gegenstand der Erfindung betrifft ein entsprechendes Verfahren zur Herstellung eines Lactosederivates mit erhöhter Süßkraft, bestehend aus oder umfassend die folgenden Schritte:
(a) Bereitstellen einer lactosehaltigen Milchfraktion;
(b) Ultrafiltration und/oder Nanofiltration der Milchfraktion unter Erhalt eines ersten protein- und fettreichen Retentats R1 und eines ersten lactosehaltigen Permeats P1;
(c) Umkehrosmose des ersten Permeats P1 unter Erhalt eines lactoseangereicherten zweiten Retentats R2 und Permeatwasser;
(d) Elektrodialyse und/oder Kationenaustauscherbehandlung des zweiten Retentats R2 unter Erhalt einer Salzfraktion und einer weiter aufkonzentrierten Lactoselösung als Diluat;
(e) erste Enzymbehandlung der aufkonzentrierten Lactoselösung unter Zugabe einer solchen Menge an Lactase, dass die Lactose ganz oder zumindest annähernd vollständig zu Glucose und Galactose gespalten wird;
(f) weitere Enzymbehandlung der so erhaltenen Glucose/Galactose-Lösung unter Zugabe von einer solchen Menge an Glucoseisomerase, dass ein Teil, vorzugsweise etwa 40 bis etwa 50 % der Glucose in Fructose umgewandelt wird; sowie gegebenenfalls
(g) Entwässerung des so erhaltenen Endproduktes.

Überraschenderweise wurde gefunden, dass sich auf diese Weise Lactosederivate auf Milchbasis zur Verfügung stellen lassen, die im gewichtsgleichen Vergleich mit Lactose über die doppelte bis 3fache Süßkraft verfügen. Durch den Austausch von Lactose in oralen Zubereitungen, wie Lebensmitteln oder auch pharmazeutischen Produkten, gegen den Ersatzstoff der vorliegenden Erfindung lässt sich die gleiche Süßkraft mit deutlich reduzierter Menge und damit entsprechend verringertem Kalorieneintrag bewerkstelligen.

### Lactosehaltige Milchfraktionen

Als lactosehaltige Milchfraktionen, die als Edukte für die Herstellung der Lactosederivate dienen, kommen insbesondere Vollmilch, Magermilch, Süßmolke und Sauermolke in Betracht. Grundsätzlich kann auch jede wässrige Lactoselösung eingesetzt werden.

### Ultra- und Nanofiltration

Ultra- und Nanofiltration sind Filtrationsverfahren aus dem Bereich der Membrantechnik, mit der sich makromolekulare Substanzen und kleine Partikel aus einem Medium abtrennen und aufkonzentrieren lassen. Man unterscheidet Mikrofiltration, Ultrafiltration und Nanofiltration über den Grad der Abtrennung. Liegt die Ausschlussgrenze (oder auch "*Cut-off*") bei 100 nm oder darüber, spricht man von Mikrofiltration. Liegt die Ausschlussgrenze in dem Bereich zwischen 2-100 nm, bezeichnet man dies als Ultrafiltration. Bei der Nanofiltration liegt die Ausschlussgrenze unterhalb von 2 nm. In jedem dieser Fälle handelt es sich um rein physikalische, d.h. mechanische Membrantrennverfahren, die nach Prinzip des mechanischen Größenausschlusses arbeiten: alle Partikel in den Fluiden, die größer als die Membranporen sind, werden von der Membran zurückgehalten. Treibende Kraft in beiden Trennverfahren ist der Differenzdruck zwischen Zulauf und Ablauf der Filterfläche, der zwischen 0,1 und 10 bar liegt.

Die Ausschlussgrenzen von Ultrafiltrationsmembranenen werden auch in Form des *NMWC* (englisch: Nominal Molecular Weight Cut-Off, auch *MWCO,* Molecular Weight Cut Off, Einheit: Dalton) angegeben. Er ist definiert als die minimale Molekülmasse globulärer Moleküle, welche durch die Membran zu 90% zurückgehalten werden. In der Praxis sollte der NMWC mindestens 20 % niedriger sein als die Molmasse des abzutrennenden Moleküls. Weitere qualitative Aussagen über die Filtration lassen sich anhand des *Flux* (Wasserwert) (Transmembranfluss oder Durchtrittsrate) machen. Dieser verhält sich im Idealfall proportional zum Transmembrandruck und reziprok zum Membranwiderstand. Diese Größen werden sowohl von den Eigenschaften der verwendeten Membran als auch durch Konzentrationspolarisation und eventuell auftretendes Fouling bestimmt. Die Durchtrittsrate wird auf 1 m² Membranfläche bezogen. Ihre Einheit ist l/(m²h bar).

Für die Ultrafiltration haben sich Membranen als besonders geeignet erwiesen, die einen Porendurchmesser im Bereich von etwa 1.000 bis etwa 50.000 und vorzugsweise etwa 5.000 bis etwa 25.000 Dalton besitzen. Die Nanofiltration bevorzugt Porendurchmesser im Bereich von 100 bis 5.000 und vorzugsweise etwa 500 bis 2.000 Dalton.

Der Werkstoff der Filterfläche - sowohl bei der Ultra- als auch der Nanofiltration - kann Edelstahl, Polymerwerkstoffen, Keramik, Aluminiumoxid oder textilen Gewebe darstellen. Es gibt verschiedene Erscheinungsformen der Filterelemente: Kerzenfilter, Flachmembranen, Spiralwickelmembranen, Taschenfilter und Hohlfasermodule, die im Sinne der vorliegenden Erfindung alle grundsätzlich geeignet sind. Vorzugsweise werden jedoch Spiralwickelmembranen aus Polymerwerkstoffen oder Kerzenfilter aus Keramik oder Aluminiumoxid eingesetzt, wobei sich die erste Ausführungsform für die Ultrafiltration und die zweite für die Nanofiltration besonders bevorzugt erwiesen hat.

Sowohl Ultra- als auch Nanofiltration können im Sinne der vorliegenden Erfindung "heiß" oder "kalt", d.h. im Temperaturbereich von etwa 10 bis etwa 60 °C durchgeführt werden. Bevorzugt ist es jedoch, bei Temperaturen im niedrigen Bereich von etwa 10 bis etwa 20 °C zu arbeiten.

Im Sinne der vorliegenden Erfindung ist die Durchführung einer Ultrafiltration bevorzugt.

### Umkehrosmose

Die Umkehrosmose oder Reversosmose ist ein physikalisches Membranverfahren zur Konzentrierung von in Flüssigkeiten gelösten Stoffen, bei der mit Druck der natürliche Osmose-Prozess umgekehrt wird.

Das Verfahrensprinzip besteht darin, dass das Medium, in dem die Konzentration eines bestimmten Stoffes verringert werden soll, durch eine halbdurchlässige (semipermeable) Membran von dem Medium getrennt ist, in dem die Konzentration erhöht werden soll. Dieses wird einem Druck ausgesetzt, der höher sein muss als der Druck, der durch das osmotische Verlangen zum Konzentrationsausgleich entsteht. Dadurch können die Moleküle des Lösungsmittels gegen ihre "natürliche" osmotische Ausbreitungsrichtung wandern. Das Verfahren drückt sie in das Kompartiment, in dem gelöste Stoffe weniger konzentriert vorliegen. Typische Drücke für die Umkehrosmose liegen im Bereich von 3 bis 30 bar. Der Konzentrierungsfaktor - entsprechend der Menge an verbliebenem Wasser im Konzentrat und der Ausgangsmenge - kann dabei zwischen etwa 2 und etwa 10, vorzugsweise etwa 5 und etwa 50 und insbesondere 10 bis etwa 25 liegen.

Die osmotische Membran, die nur die Trägerflüssigkeit (Solvent) durchlässt und die gelösten Stoffe (Solute) zurückhält, muss diesen hohen Drücken standhalten können. Wenn der Druckunterschied das osmotische Gefälle mehr als ausgleicht, passen die Solventmoleküle wie bei einem Filter durch die Membran, während die "Verunreinigungsmoleküle" zurückgehalten werden. Im Gegensatz zu einem klassischen Membranfilter verfügen Osmosemembranen nicht über durchgehende Poren. Vielmehr wandern die Ionen und Moleküle durch die Membran hindurch, indem sie durch das Membranmaterial diffundieren, wie dies durch das Lösungs-Diffusions-Modell beschrieben wird: Der osmotische Druck steigt mit zunehmendem Konzentrationsunterschied. Wird der osmotische Druck gleich dem angelegten Druck, kommt der Prozess zum Stehen. Es liegt dann ein osmotisches Gleichgewicht vor. Ein stetiger Abfluss des Konzentrats kann das verhindern. Beim Konzentratauslass wird der Druck entweder über einen Druckregler kontrolliert oder über einen Druckaustauscher genutzt, um den im Zulauf des Systems benötigten Druck aufzubauen.

### Elektrodialyse

Hierbei handelt es sich um einen elektrochemisch getriebenen Membranprozess, in dem Ionenaustauschermembranen in Kombination mit einer elektrischen Potentialdifferenz benutzt werden, um ionische Spezies- also vor allem Salze - von ungeladenen Lösungsmitteln oder Verunreinigungen abzutrennen.

Dazu wird in einem Elektrodialyse-Separator der Raum zwischen zwei Elektroden durch einen Stapel aus einander abwechselnden Anionen- und Kationenaustauschermembranen getrennt. Jedes Paar Ionenaustauschermembranen bildet eine separate "Zelle". In technischen Systemen bestehen diese Stapel aus mehr als zweihundert Membranpaaren. Wird eine elektrische Gleichspannung an die Elektroden angelegt, so wandern die Anionen zur Anode. Die Anionen können einfach die positiv geladenen Anionenaustauschermembranen passieren, aber sie werden jeweils an der nächstgelegenen negativ geladenen Kationenaustauschermembran gestoppt. Weil dasselbe (natürlich mit umgekehrten Vorzeichen) auch mit den Kationen geschieht, besteht der Nettoeffekt der Elektrodialyse in einer Anreicherung der Salze in den Zellen mit ungerader Nummer (Anionentauschermembran/Kationenaustauschermembran), während die Zellen mit gerader Nummer (Kationenaustauschermembran/Anionentauschermembran) an Salz verarmen. Die Lösungen mit erhöhter Salzkonzentration werden zum Konzentrat vereint, während die salzarmen Lösungen das Diluat bilden. Es empfiehlt sich, dass man das Diluat abschließend mit einem Kationenaustauscher ("Polisher") behandelt.

Anstelle oder zusätzlich zu einer Elektrodialyse kann auch eine Behandlung mit einem handelsüblichen Kationenaustauscher durchgeführt werden.

### Enzymatische Behandlung

Die beiden Enzymbehandlungsschritte dienen dazu, Lactose zunächst möglichst vollständig in Glucose und Galactose zu spalten und den Glucoseanteil anschließend zu etwa 40 bis etwa 50 % in Fructose umzulagern.

Lactose gehört zur Gruppe der Disaccharide und besteht aus den beiden Molekülen D-Galactose und D-Glucose, die über eine β-1,4-glycosidische Bindung verbunden sind.

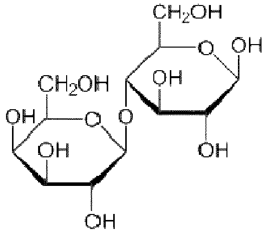

Zum Abbau in die beiden Zuckerkomponenten wird Lactose mit dem Enzym Lactase (auch als LPH oder *LCT* bezeichnet) versetzt. Die Hydrolyse erfolgt vorzugsweise in einem Rührbehälter mit kontinuierlichem Zulauf und Ablauf sowie einer Dosiervorrichtung zur Zugabe des Enzyms und einem am Boden des Reaktors befindlichen Ventil zum Ablassen desaktivierten Enzyms, das im Laufe der Zeit sedimentiert. Es hat sich als vorteilhaft erwiesen, eine wirksame Enzymkonzentration von etwa 180.000 bis 250.000 FCC-Einheiten Lactase pro kg zu hydrolysierender Lactose einzusetzen und die Reaktion bei Temperaturen im Bereich von etwa 23 bis etwa 27 °C sowie einem leicht sauren pH-Wert von etwa 5 bis 6 durchzuführen. Mit diesen Bedingungen ist es möglich, mindestens 90 Mol-%, vorzugsweise etwa 95 bis 98 Mol.-% der vorhandenen Lactose zu spalten

Der Reaktor kann aber auch mit einer NF-Einheit verbunden sein, so dass die gesamte Reaktionsmischung ständig über die Membran im Kreis geführt wird. Über die Permeatseite wird lactosefreie Lösung abgegeben, die dann in den Vorratsbehälter geleitet und mit dem Retentat aus der UF-Einheit vermischt wird, während das Retentat in den Reaktor zurückgeführt wird. Enzym geht auf diese Weise nicht verloren, lediglich muss verbrauchter Katalysator entweder kontinuierlich oder batchweise zugeführt werden, um eine möglichst gleichmäßige Enzymkonzentration zu gewährleisten. Inaktives Enzym sinkt im Laufe des Prozesses zu Boden und kann dann bei kurzer Unterbrechung des Verfahrens durch ein Bodenventil abgelassen werden. Ein solcher Reaktor ist Gegenstand der EP 2907393 A1 (DMK).

Die partielle Umlagerung der Glucose in Fructose erfolgt durch Zugabe von Glucoseisomerase. Dabei setzt man der Lösung eine solche Menge des Enzyms zu, die ausreicht etwa 40 bis etwa 60 Mol.-% der vorhandenen Glucose in Fructose umzulagern. Alternativ - oder zusätzlich - ist es möglich, die Umlagerung, bei der es sich um eine Aldose-Ketose-Tautomerie handelt, auch über den pH-Wert zu steuern. Dies ist als Lobry-de-Bruyn-Alberda-van-Ekenstein-Umlagerung bekannt.

Es ist dabei möglich, die beiden entweder einzeln oder kombiniert kontinuierlich durchzuführen; ein geeigneter Reaktor ist oben beschrieben.

Typisch weisen die resultierenden Lactosederivate - bezogen auf die Trockenmasse - folgende Zusammensetzung auf:
(a) etwa 20 bis etwa 30 Gew.-%, vorzugsweise etwa 22 bis etwa 26 Gew.-% Glucose,
(b) etwa 40 bis etwa 60 Gew.-%, vorzugsweise etwa 45 bis etwa 55 Gew.-% Galactose sowie
(c) etwa 20 bis etwa 30 Gew.-%, vorzugsweise etwa 22 bis etwa 26 Gew.-% Fructose,
mit der Maßgabe, dass sich die Mengenangaben jeweils zu 100 Gew.-% ergänzen. Zur Vermeidung von Unklarheiten sei darauf hingewiesen, dass Gemische, deren Mengen sich nicht zu 100 Gew.-% ergänzen oder über 100 Gew.-% hinausgehen nicht beansprucht werden und der Fachmann ohne weiteres in der Lage ist, nur anspruchskonforme Mischungen zur Lösung der Aufgabe heranzuziehen.

### Trocknung

In einem optionalen Verfahrensschritt kann das Produkt anschließend getrocknet werden. Vorzugsweise kommt dabei die Sprühtrocknung zum Zuge, wobei die Temperatur im Einlass typischerweise etwa 180 bis etwa 260 °C und am Ausgang etwa 80 bis etwa 105 °C beträgt. Die Fraktion bedarf daher keiner Kühlung bevor sie in den Sprühturm gelangt. Temperaturen von 60 bis 70 °C sind dabei sogar bevorzugt, da auf diese Weise die Gefahr verringert wird, dass die Kohlenhydrate sich verfärben. Alternativ können die Produkte auch durch Gefriertrocknung entwässert werden. Der Restwassergehalt beträgt dabei in beiden Fällen maximal 5 Gew.-% und vorzugsweise etwa 2 bis etwa 4 Gew.-%.

### GEWERBLICHE ANWENDBARKEIT

Ein weiterer Gegenstand der Erfindung betrifft ein Verfahren zur Verstärkung des Süßeindrucks einer oralen Zubereitung, umfassend die Schritte
(i) Bereitstellen der oralen Zubereitung und
(ii) Vermischen der oralen Zubereitung mit einer wirksamen Menge der Lactosederivate wie vorstehend beschrieben.

Ebenfalls beansprucht wird zum einen die Verwendung der Lactosederivate wie oben beschrieben als Substitut für Saccharose sowie als Süßstoff für orale Zubereitungen, wie insbesondere Nahrungsmittel, pharmazeutische Produkte, wie etwa Hustensaft oder Erkältungsbonbons oder Kaugummis sowie orale Zubereitungen, die den Lactosederivat, beispielsweise in Mengen von etwa 0,5 bis etwa 2 Gew.-% enthalten.

### Nahrungsmittel

Beispiele für Nahrungsmittel, denen die erfindungsgemäßen Lactosederivate zugegeben werden können, umfassen Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

### Kaugummis

Kaugummis, die die erfindungsgemäßen Lactosederivate enthalten, weisen stets eine wasserunlösliche und eine wasserlösliche Komponente auf.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Lactosederivate kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Süßstoffe und Süßstoffverstärker

Ein weiterer Gegenstand der Erfindung betrifft Süßstoffe oder Süßkraftverstärker enthaltend oder bestehend aus
(a) dem Lactosederivat wie vorstehend beschrieben sowie
(b) wenigstens einem Süßstoff ausgewählt aus der Gruppe, die gebildet wird von Rebaudiosiden, insbesondere Rebaudiosid D, Steviosiden, Naringin, Dihydrochalconen, Mogrosiden, Rubus-Extrakten sowie Glycyrrhizinsäure und deren Salzen.

**Rebaudioside** gehören zu den Steviosiden, den Hauptbestandteilen der Pflanze *Stevia rebaudiana*, die auch als Süßkraut oder Honigkraut bezeichnet wird.

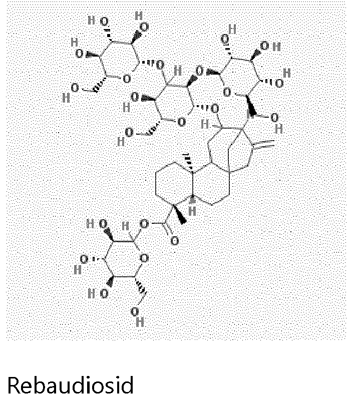

10 % der Trockenmasse der Blätter macht das Diterpenglykoid Steviosid aus, gefolgt von Rebaudiosid A (2 bis 4 Gew.-%) sowie zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Besonders bevorzugt ist jedoch die Verwendung von Rebaudiosid A, da dieser Stoff eine geringere Bitterkeit und die höchste Süßkraft aufweist. Die erfindungsgemäßen Stoffgemische können die Komponenten (a) und (b) im Gewichtsverhältnis von etwa 1:99 bis etwa 99:1, vorzugsweise etwa 25:75 bis etwa 75:25 und insbesondere etwa 40:60 bis etwa 60:40 enthalten

Alternativ oder ergänzend zu den Rebaudiosiden bzw. Stevia Extrakten können als Komponente (c) auch verschiedene andere Wirkstoffe eingesetzt werden, die ebenfalls die Zitrusnote verstärken, selbst aber kein Fruchtaroma aufweisen. Es sind dies:
- Naringin,
- Dihydrochalcone,
- Mogroside sowie
- Extrakten der Pflanzen der Gattung *Rubus.*

**Naringin** ist ein polyphenolisches Glykosid, das in der Grapefruit und dem Pomelo enthalten ist und diesen einen bitteren Geschmack verleiht. Der Stoff ist insbesondere wegen seiner lipidsenkenden Wirkung bekannt.

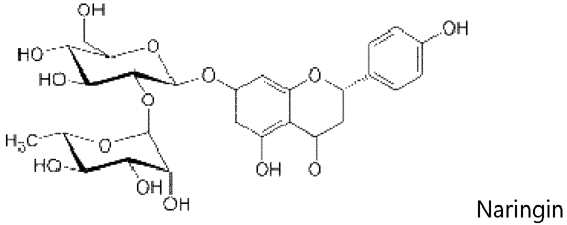

Auch die **Dihydrochalcone** stellen Polyphenole dar, wobei insbesondere die beiden Vertreter Dihydrochalcon und Neohesperidin Dihydrochalcon hervorzuheben sind, die als künstliche Süßungsmittel bekannt sind:

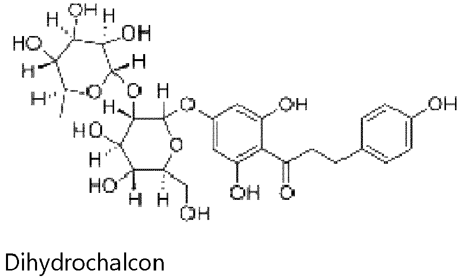

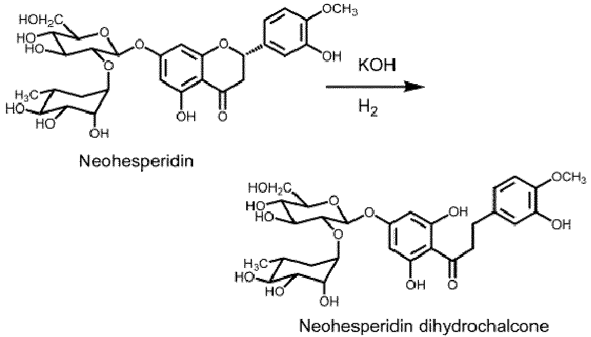

Unter der Bezeichnung **Mogroside** wird eine Gruppe von Glykosiden des Cucurbitans verstanden, die als Bestandteil des natürlichen Süßungsmittels Luo Han Guo bekannt sind. Hervorzuheben ist hier das Mogrosid-5, das 400mal süßer als Zucker ist.

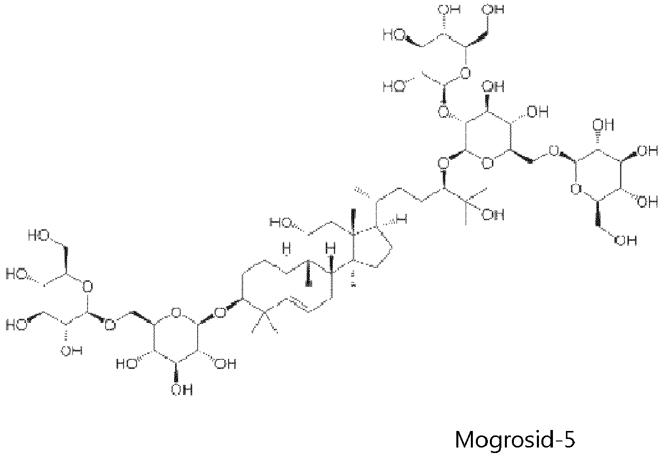

Schließlich kommen als Komponente (c) Extrakte der Pflanzen in Betracht, die ausgewählt sind aus der Gruppe, die gebildet wird von *Rubus allegheniensis*, *Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus, Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis* und *Rubus ulmifolius* sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von *Rubus Suavissimus.*

Ein weiterer Wirkstoff in dieser Gruppe ist die **Glycyrrhizinsäure** oder ein entsprechendes Salz oder einen Extrakt, der diesen Stoff enthält.

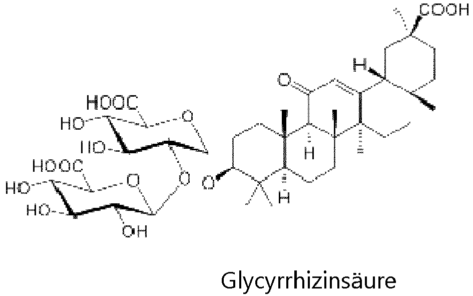

Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

Die Mischungen können die Komponenten (a) und (b) im Gewichtsverhältnis von etwa 1:99 bis etwa 99:1, vorzugsweise von etwa 10:90 bis etwa 90:10, insbesondere von etwa 25:75 bis etwa 75:25 und besonders bevorzugt von etwa 40:60 bis etwa 60:40 enthalten. Insbesondere mit Rebaudiosid D beobachtet man eine synergistische Verstärkung der Süßkraft sowie eine Maskierung des metallischen Beigeschmacks.

### BEISPIELE

### Beispiel 1

### Herstellung einer Kohlenhydratmischung

1.000 L Vollmilch wurde bei 8 °C einer Ultrafiltration mit einer Spiralwickelmembran (Porendurchmesser: 40.000 Dalton) unterworfen. Das lactosereiche Permeat wurde auf eine Umkehrosmoseanlage gegeben und mit dem Faktor 5 aufkonzentriert. Das Permeatwasser wurde abgetrennt und das Lactosekonzentrat einer Elektrodialyse unterworfen und entsalzt. Dem Diluat wurde pro kg darin enthaltener Lactose 200.000 FCC-Einheiten Lactase zugesetzt und die Mischung bei 25 °C und pH = 5,5 für 3 h hydrolysiert, wobei 98 Mol.-% der Lactose in Glucose und Galactose gespalten wurden. Der Mischung wurden 0,1 Gew.-% Glucoseisomerase zugesetzt und die Mischung weitere 3 h gerührt. Anschließend wurde das Reaktionsprodukt auf einen Sprühturm gegeben und bei einer Temperatur von etwa 150 °C entwässert. Es wurde ein farbloses Pulver erhalten, welches 23 Gew.-% Glucose, 23 Gew.-% Fructose, 53 Gew.-% Galactose und 1 Gew.-% Lactose aufwies.

### Beispiele 1 und 2, Vergleichsbeispiele V1 bis V3

### Verkostung

Es wurden 10 bzw. 0,01 Gew.-%ige Lösungen verschiedener Süßstoffe in Leitungswasser normaler Härte hegestellt und von einem Panel bestehend aus 10 erfahrenen Testern bezüglich Süßkraft (10 = sehr süß; 1 = kaum Süßeffekt) und Beigeschmack beurteilt. Die Ergebnisse sind in Tabelle 1 wiedergegeben.

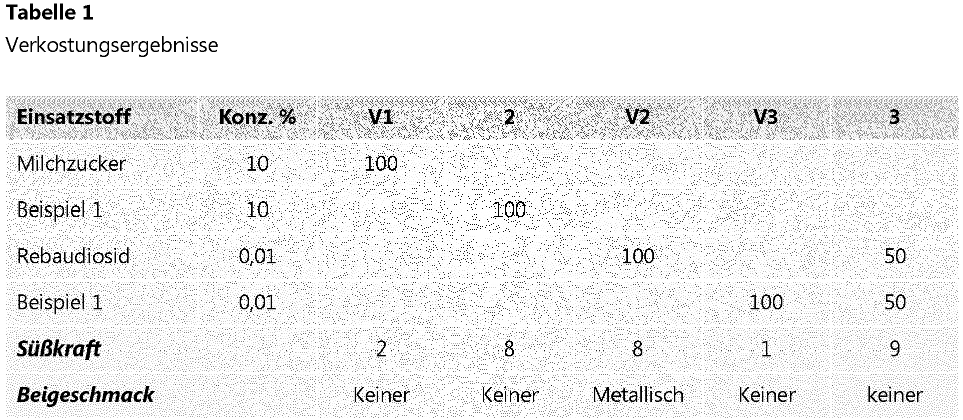

Die Beispiele und Vergleichsbeispiele zeigen zum einen, dass das erfindungsgemäße Lactosederivat gegenüber Lactose über eine 2-3fach höhere Süßkraft verfügt und zum anderen sowohl die Süßkraft von Rebaudiosid D synergistisch verstärkt, als auch dessen unangenehmen metallischen Beigeschmack maskiert.

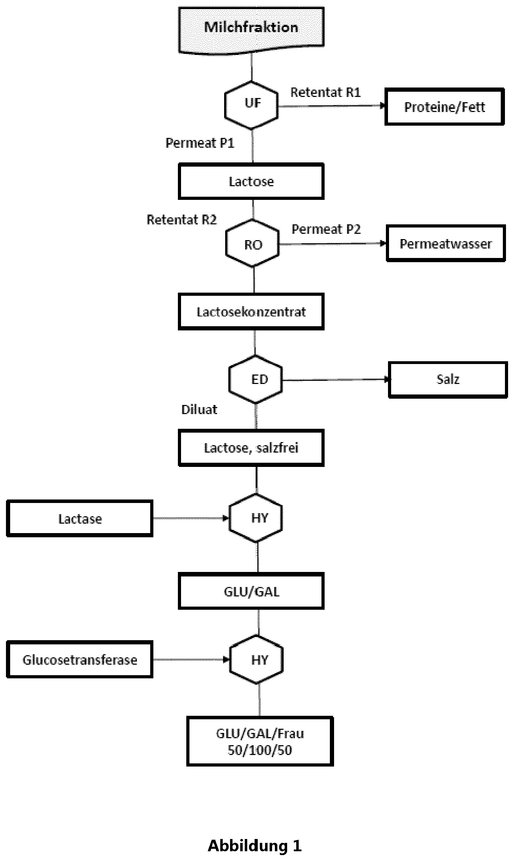

Die Herstellung der erfindungsgemäßen Lactosederivate wird exemplarisch durch das Fließschema gemäß **Abbildung 1** wiedergegeben. Die Abkürzungen bedeuten:
UF = Ultrafiltration
RO = Umkehrosmose
ED = Elektrodialyse
HY = Hydrolyse

## Patentansprüche

1. Lactosederivat mit erhöhter Süßkraft, dadurch erhältlich oder erhalten, dass man
(a) eine lactosehaltige Milchfraktion bereitstellt;
(b) die Milchfraktion einer Ultrafiltration und/oder Nanofiltration unterwirft und dabei ein erstes protein- und fettreiches Retentat R1 und ein erstes lactosehaltiges Permeat P1 erhält;
(c) das erste Permeat P1 einer Umkehrosmose unterwirft und dabei ein lactoseangereichertes zweites Retentat R2 und Permeatwasser erhält;
(d) das zweite Retentat R2
(d1) einer Elektrodialyse und/oder
(d2) einer Kationenaustauscherbehandlung
unterwirft und dabei eine Salzfraktion und als Diluat eine weiter aufkonzentrierte Lactoselösung erhält;
(e) die aufkonzentrierte Lactoselösung in einer ersten Enzymbehandlungsstufe mit Lactase versetzt und die Lactose vollständig oder zumindest annähernd vollständig zu einem Gemisch von Glucose und Galactose umsetzt;
(f) die so erhaltene Glucose/Galactose-Lösung in einer zweiten Enzymbehandlungsstufe mit Glucoseisomerase versetzt und dabei einen Teil der Glucose in Fructose umlagert; sowie gegebenenfalls
(g) das so erhaltene Endprodukt entwässert.

2. Lactosederivat nach Anspruch 1, **dadurch gekennzeichnet, dass** seine Süßkraft mindestens doppelt so hoch ist wie die Süßkraft einer gewichtsgleichen Menge an Milchzucker.

3. Verfahren zur Herstellung eines Lactosederivates mit erhöhter Süßkraft, bestehend aus oder umfassend die folgenden Schritte:
(a) Bereitstellen einer lactosehaltigen Milchfraktion;
(b) Ultrafiltration und/oder Nanofiltration der Milchfraktion unter Erhalt eines ersten protein- und fettreichen Retentats R1 und eines ersten lactosehaltigen Permeats P1;
(c) Umkehrosmose des ersten Permeats P1 unter Erhalt eines lactoseangereicherten zweiten Retentats R2 und Permeatwasser;
(d) Elektrodialyse oder Kationenaustauscherbehandlung des zweiten Retentats R2 unter Erhalt einer Salzfraktion und einer weiter aufkonzentrierten Lactoselösung als Diluat;
(e) erste Enzymbehandlung der aufkonzentrierten Lactoselösung unter Zugabe einer solchen Menge an Lactase, dass die Lactose ganz oder zumindest annähernd vollständig zu Glucose und Galactose gespalten wird;
(f) weitere Enzymbehandlung der so erhaltenen Glucose/Galactose-Lösung unter Zugabe von einer solchen Menge an Glucoseisomerase, dass ein Teil der Glucose in Fructose umgewandelt wird; sowie gegebenenfalls
(g) Entwässerung des so erhaltenen Endproduktes.

4. Verfahren nach Anspruch 3, **dadurch gekennzeichnet, dass** man als lactosehaltige Milchfraktion Vollmilch, Magermilch, Süßmolke, Sauermolke oder eine wässrige Lactoselösung einsetzt.

5. Verfahren nach den Ansprüchen 3 und/oder 4, **dadurch gekennzeichnet, dass** man eine Ultrafiltration mit einer Membran durchführt, die einen mittleren Porendurchmesser von etwa 1.000 bis etwa 50.000 Dalton aufweisen.

6. Verfahren nach mindestens einem der Ansprüche 3 bis 5, **dadurch gekennzeichnet, dass** man die Umkehrosmose mit einem Konzentrierungsfaktor von etwa 2 bis etwa 10.

7. Verfahren nach mindestens einem der Ansprüche 3 bis 6, **dadurch gekennzeichnet, dass** man die erste Hydrolyse in Gegenwart einer solchen Menge an Lactase und unter solchen Bedingungen durchführt, dass mindestens 90 Mol.-% der vorhandenen Lactose in Glucose und Galactose gespalten wird.

8. Verfahren nach mindestens einem der Ansprüche 3 bis 7, **dadurch gekennzeichnet, dass** man die zweite Hydrolyse in Gegenwart einer solchen Menge an Glucoseisomerase und unter solchen Bedingungen durchführt, dass etwa 40 bis etwa 60 Mol.-% der vorhandenen Glucose in Fructose umgelagert wird.

9. Verfahren nach mindestens einem der Ansprüche 3 bis 9, **dadurch gekennzeichnet, dass** man die Hydrolyseschritte entweder einzeln oder kombiniert kontinuierlich durchführt.

10. Verfahren nach mindestens einem der Ansprüche 3 bis 10, **dadurch gekennzeichnet, dass** man die wässrige Lösung enthaltend Glucose, Galactose und Fructose durch Sprühtrocknung oder Gefriertrocknung bis auf einen Restwassergehalt von maximal 5 Gew.-% entwässert.

11. Verfahren zur Verstärkung des Süßeindrucks einer oralen Zubereitung, umfassend die Schritte
(i) Bereitstellen der oralen Zubereitung und
(ii) Vermischen der oralen Zubereitung mit einer wirksamen Menge des Stoffes nach Anspruch 1.

12. Verwendung des Stoffes nach Anspruch 1 als Substitut für Saccharose.

13. Verwendung des Stoffes nach Anspruch 1 als Süßstoff für orale Zubereitungen.

14. Orale Zubereitungen enthaltend den Stoff nach Anspruch 1.

15. Süßstoff oder Süßkraftverstärker enthaltend oder bestehend aus
(a) dem Lactosederivat nach Anspruch 1 und
(b) wenigstens einem Süßstoff ausgewählt aus der Gruppe, die gebildet wird von Rebaudiosiden, insbesondere Rebaudiosid D, Steviosiden, Naringin, Dihydrochalconen, Mogrosiden, Rubus-Extrakten sowie Glycyrrhizinsäure und deren Salzen.
